# EUROPÄISCHE PATENTSCHRIFT

(11) **EP 3 387 916 B1**
(45) Veröffentlichungstag und Bekanntmachung des Hinweises auf die Patenterteilung: **05.08.2020**
(21) Anmeldenummer: 18167143.9
(22) Anmeldetag: 13.04.2018
(51) Int. Cl.: A23L 33/105, A23L 33/11, A23L 21/10, A23L 19/00, A23P 20/10, A23P 10/30, A23P 10/35, A61K 36/00, A61K 36/482, A61K 36/68, A61K 36/708, A61P 1/10

(54) **ESSBARE ZUSAMMENSETZUNG ZUR VERDAUUNGSFÖRDERUNG**
EDIBLE COMPOSITION FOR DIGESTIVE PROMOTION
COMPOSITION COMESTIBLE POUR PROMOTION DIGESTIV

(30) Priorität: 13.04.2017 DE 102017108054
(43) Veröffentlichungstag der Anmeldung: 17.10.2018
(73) Patentinhaber: Natura-Werk Gebr. Hiller GmbH & Co. KG, 30165 Hannover (DE)
(72) Erfinder: Gooß, Thorsten, 27753 Delmenhorst (DE); Schwetje, Norbert, 30539 Hannover (DE)
(74) Vertreter: Held, Stephan

(56) Entgegenhaltungen:
- WO-A1-95/17201
- WO-A1-2013/067424
- DE-A1-102005 005 276
- FR-A1- 2 922 768
- US-A- 4 476 121
- US-A- 4 511 561
- US-A- 5 232 699
- US-A1- 2014 127 351
- DATABASE FSTA [Online] INTERNATIONAL FOOD INFORMATION SERVICE (IFIS), FRANkFURT-MAIN, DE; 1986, TREPTOW H: "Rhubarb (Rheum sp.) and its use. (translated) TIOL- Rhabarber (Rheumarten) und seine Verwendung.", XP002781812, Database accession no. FS-1986-09-J-0187 & ERNÄHRUNG, Bd. 9, Nr. 3, 1985, Seiten 179-183,

## Beschreibung

Die Erfindung betrifft essbare Formulierungen, die mindestens einen mit einer niedermolekularen hydrophoben Substanz beschichteten partikulären pflanzlichen Stoff enthalten, wobei der pflanzliche Stoff Wirkstoffe zur Verdauungsförderung enthält. Für den Einbezug in die erfindungsgemäßen Formulierungen geeignete pflanzliche Stoffe sind insbesondere Teile von Sennespflanzen, wie Blätter oder Samen, Plantagosamen oder Rhabarberwurzeln, die gegenüber der Magensäure instabile Wirkstoffe enthalten. Die Erfindung betrifft ebenso entsprechende essbare Formulierungen zur Verwendung als Abführmittel und die Verwendung derselben als Brotaufstrich oder Nahrungsergänzungsmittel.

### Stand der Technik

Früchte, wie Pflaumen, Artischocken oder Feigen, sind für ihre Wirkung als stimulierende Abführmittel bekannt. Um diese Wirkung zu verbessern wurden im Stand der Technik Früchtewürfel aus den genannten Basismaterialien mit Zusätzen von Sennesblätterpulver oder Sennesfrüchtepulver oder Plantagosamen modifiziert, um Feinkostartikel oder Abführmittel herzustellen.
Sennesblätter werden seit dem Mittelalter als Arzneistoff verwendet. Seit dieser Zeit bilden sie ein beliebtes und häufig benutztes Abführmittel in der Anwendung als Aufguss, als Pulver, als Auszug (Extrakt) oder in Form von Tabletten, Granulaten und Kapseln. Neben Sennesblättern gehören auch Sennesfrüchte zu den häufig verwendeten pflanzlichen Abführmitteln.

Die für die abführende Wirkung verantwortlichen Inhaltsstoffe der Sennespflanzen, die Anthraglykoside, werden erst im Dickdarm aktiv. Dabei wurde beobachtet, dass Sennesfrüchte im Vergleich zu den Blättern eine mildere Wirkung haben.

Heute werden im Wesentlichen zwei Sennesarten arzneilich genutzt, bei denen es sich um den Tinnevelly-Sennes (Cassia angustifolia) und den Alexandriner-Sennes (Cassia senna) handelt. Die schonend getrockneten Früchte der beiden Pflanzen unterscheiden sich im Gehalt ihres Hauptinhaltsstoffes, des Anthraglykosids. Sennesschoten und -blätter enthalten die wirksamen Anthranoide, die in der Pflanze mit Zuckermolekülen verknüpft sind. Die Anthranoide setzen sich aus den Substanzen Sennosid A bis F zusammen. Die mit den Zuckermolekülen verknüpften Substanzen gelangen beim Menschen bis in den Dickdarm und werden dort von Bakterien aufgespalten. Dabei wird die aktive Form der Anthranoide freigesetzt, die bewirkt, dass weniger Wasser und Salze über die Darmschleimhaut aufgenommen werden. Dadurch reichert sich im Verdauungstrakt die salzhaltige Flüssigkeit vermehrt an, was die Darmentleerung beschleunigt.

Ferner sind Sennosid-Molekularkomplexe mit Polysacchariden, Sennosid A und B mit Guarharz, Polygalacturonsäure oder Alginsäure als therapeutische Mittel bekannt, die die peristaltische Bewegung anregen und daher für die Behandlung von Verstopfungen verwendet wird.

Plantagosamen (Plantago ovata), die auch unter der Bezeichnung Flohsamen als Heilpflanze bekannt sind, werden hauptsächlich in Indien und Pakistan angebaut. Flohsamen helfen bei Reizdarmsyndrom und vielen anderen Beschwerden rund um den Magen-Darm-Trakt und sind insbesondere bei Verwendung als Arzneimittel zur Regulierung der Darmtätigkeit bekannt. Die Samen besitzen eine beträchtliche Quellfähigkeit und üben einen physikalischen Dehnungsreiz auf die empfindlichen Rezeptoren der Darmwände aus.

Bekannt ist weiterhin, dass die Rhabarberwurzel (Rheum palmatum und Rheum officinale) wirksamkeitsbestimmenden Inhaltsstoffe, die sogenannten Anthracenderivate mit den zugehörigen Anthranoide enthalten. Anthracenderivate haben generell einen abführenden Effekt. Sie wirken direkt auf die Muskulatur des Darms und erhöhen seine Bewegungstätigkeit.

Im Stand der Technik wurden auch Mischungen aus vermahlenen Plantagosamen und Sennafrüchten beschrieben, die in verschiedenen Maischvorgängen mit Wasser konfektioniert werden.

Obwohl die kräftig verdauungsfördernde Wirkung der Sennoside allgemein geschätzt wird, gibt es Nachteile, die den Bereich ihrer Anwendbarkeit einengen. So sind die Sennoside in wässrigen Flüssigkeiten relativ unlöslich, so dass sich stabile wässrige Zubereitungsformen mit geeigneten Konzentrationen an aktiven Sennosiden nicht herstellen lassen. Darüber hinaus besteht die Schwierigkeit, dass die Sennoside, bei denen es sich um Verbindungen mit funktionellen Phenol-oder Polyphenolgruppen handelt, schon nach kurzer Zeit durch Einwirkung von Wasser und in Gegenwart von Sauerstoff zu einem wesentlichen Anteil abgebaut werden. Dieser hydrolytische Abbau resultiert in einer beträchtlichen Abwandlung des physiologischen Effekts.

Bisher verwendete Strategien zur Vermeidung des hydrolytischen Abbaus beinhalten die Verwendung von Sennosid enthaltenden Präparaten in trockener Form, wie als Pulver, Tabletten, Granulaten und Kapseln, die entweder aus dem isolierten aktiven Glykosid oder aus Pflanzenextrakt hergestellt werden.

In der DE 11 2010 003 201 wird eine verhältnismäßig stabile Form eines Sennesextrakt-enthaltenden Granulats beschrieben, das mit anionischen Copolymeren von Methacrylsäure und Methylmethacrylat beschichtet wird. Dieses Granulat kann z.B. in eine Geleearzneiform eingebracht werden. Da die verwendeten anionischen Copolymere in Magensaft und in reinem Wasser unlöslich, kann das Granulat im Wesentlichen unzersetzt den Magen passieren. In neutralen bis alkalischen Medium des Darms löst sich das Granulat dann auf und gibt den enthaltenen Sennesextrakt frei, so dass dieser dort seine Wirkung entfalten kann. Nachteilig an dem in der DE 11 2010 003 201 beschriebenen Granulat ist jedoch, dass für dessen Herstellung vergleichsweise große Mengen an Copolymer benötigt werden (etwa 27 Gew.-%), und dass die Herstellung der Pellets als sehr aufwendig beschrieben wird.

In der DE 602 11 130 ist ein mit einem hydrophoben und einem hydrophilen Matrixbildner formulierter Arzneimittelwirkstoff beschreiben, bei dem der Wirkstoff zunächst in der Schmelze in den Matrixbildnern gelöst und die Mischung anschließend zu einem Pulver verarbeitet wird. Durch diese Maßnahme soll eine modifizierte Freisetzung erreicht werden, während aus dem Pulver hergestellte Tabletten im Gengensatz zu konventionellen Gelmatrixtabletten leicht teilbar sind.

Die US 4,511,561 beschreibt abführende Mittel auf der Basis von trockenen Sennafrüchten und gemahlenen Plantagosamen, bei denen beide Bestandteile unter Zusatz einer geringen Wassermenge miteinander granuliert werden.

Die US 5,232,699 beschreibt abführende Mittel umfassend Plantagosamen und Sennoside, die in einem mundgerechten Fett dispergiert sind, dass eine Schmelztemperatur im Bereich von 30°C bis 50°C aufweist. Beispielhaft wird eine Beschichtungsformulierung aus etwa 99% Fett und etwa 1 % Sennakonzentrat offenbart.

Die FR2922768 beschreibt ein Verfahren zur Geschmacksmaskierung von Rhabarbertrockenprodukten, bei dem das Rhabarberprodukt mit einem wenig oder nicht wasserlöslichen Mittel beschichtet wird.

Die WO 95/17201 beschreibt ein abführendes Mittel, dass Fasern und Dioctylsulfosuccinat als Wirkstoff enthält. Zum Maskierung des Empfindens von unangenehmen Geschmack des Wirkstoffs, kann dieser mit einer Beschichtung versehen werden, die so ausgewählt sein kann, dass sie sich auflöst oder schmilzt, bevor das Mittel in den Dickdarm gelangt.

Es besteht ein Bedarf an einer Verabreichungsform für Sennesprodukte, bei der die darin enthaltenen Sennoside vor einem Kontakt mit Wasser und Sauerstoff geschützt sind, um ihre Lagerstabilität zu verbessern. Es besteht weiterhin ein Bedarf an einer Verabreichungsform von Sennesprodukten, mit denen eine gezielte Freisetzung von Sennosiden oder anderen verdauungsfördernden Stoffen im Darm ermöglicht wird. Schließlich besteht ein Bedarf an einer Verabreichungsform von Produkten mit verdauungsfördernden Inhaltsstoffen, die aus leicht verfügbaren Ausgangsmaterialien und mit minimalem Aufwand hergestellt werden können. Die vorliegende Erfindung befasst sich mit diesem Bedarf.

### Detaillierte Beschreibung der Erfindung

Gemäß einem ersten Aspekt betrifft die vorliegende Erfindung eine essbare Formulierung gemäß Anspruch 1, umfassend mindestens einen mit einer niedermolekularen hydrophoben Substanz beschichteten partikulären pflanzlichen Stoff, der Wirkstoffe zur Verdauungsförderung enthält.

In den der vorliegenden Erfindung zugrundeliegenden Untersuchungen wurde überraschend gefunden, dass sich pflanzliche Stoffe, wie Sennespulver, durch die Beschichtung mit einer niedermolekularen hydrophoben Substanz gegenüber Zersetzungseinflüssen durch Wasser oder Sauerstoff passivieren lassen, wobei eine mit der Verwendung von Acylatcopolymeren vergleichbare Stabilisierung erzielt wird.

Der Begriff "niedermolekular" ist im Kontext der vorliegenden Erfindung definiert als Molekulargewicht < 1000 g/mol. Eine niedermolekulare hydrophobe Substanz ist im Kontext der Erfindung eine Substanz oder ein Gemisch von Substanzen, deren wesentliche Inhaltsstoffe die vorstehende Molekulargewichtsvorgabe erfüllen.

Für Pflanzen wie Sennes ist bekannt, dass sie Verbindungen enthalten, die im Darm verdauungsfördernd wirken. So enthält Sennes als wirksame Verbindungen Sennoside. Für die verdauungsfördernde Wirkung kommt es daher darauf an, dass die verdauungsfördernd wirksamen Verbindungen möglichst weitgehend in den Darm gelangen. Als "pflanzlicher Stoff" werden im Rahmen dieser Erfindung daher auch Extrakte solcher Verbindungen aus dem Pflanzenmaterial und aus dem Pflanzenmaterial isolierte reine Verbindungen oder auf chemischem Wege hergestellte Verbindungen, die die gleiche chemische Struktur aufweisen, wie die aus Pflanzen extrahierten Verbindungen, bezeichnet. Eingeschlossen sind dabei auch Extrakte oder isolierte oder auf chemischem Weg hergestellte reine Verbindungen, die auf ein Trägermaterial aufgebracht wurden. Solche Trägermaterialien können als Absorptionsmittel, beispielsweise für flüssige Extrakte, oder zur besseren Konzentrationskontrolle der wirksamen Verbindungen in der Endformulierung eingesetzt werden.

Die Extrakte, die isolierten oder die auf chemischem Wege hergestellten reinen Verbindungen lassen sich analog zu partikulärem Pflanzenmaterial beschichten und so zu einem beschichteten partikulären pflanzlichen Stoff verarbeiten.

Bei vielen Naturstoffen ist die Herstellung der Stoffe auf chemischem Weg zwar möglich, die Kosten so hergestellter Stoffe können aber signifikant höher sein als bei einer Extraktion der Stoffe aus Pflanzen. Darüber hinaus ist die Akzeptanz von aus Pflanzen oder anderem biologischen Material gewonnenen Naturstoffen beim Verbraucher höher, als wenn die gleichen Naturstoffe chemisch hergestellt werden. Bevorzugt wird im Rahmen der vorliegenden Erfindung daher ein pflanzlicher Stoff eingesetzt, der aus Pflanzenmaterial besteht oder ein Extrakt oder eine reine isolierte Verbindung aus Pflanzenmaterial ist. Da zudem ein Extraktionsschritt häufig mit zusätzlichen Kosten verbunden ist, ist die Verwendung eines pflanzlichen Stoffs, der aus Pflanzenmaterial besteht, im Rahmen der vorliegenden Erfindung besonders bevorzugt. Als meist bevorzugte pflanzliche Stoffe sind pulverisierte oder partikuläre pflanzliche Stoffe, wie schlichtes Sennesblattpulver, zu nennen, die günstig am Markt verfügbar sind und keine weitere Aufbereitung erfordern.

Der pflanzliche Stoff in der angegebenen essbaren Formulierung liegt in Form von Sennes, insbesondere in Form von Sennesblätter, Sennesextrakt, reinen Sennosiden, Rhabarberwurzeln oder Rhabarberwurzelextrakt vor. Als im Kontext der vorliegenden Erfindung besonders bevorzugte pflanzliche Stoffe sind Sennes (Cassia angustifolia oder Cassia senna), Plantagosamen (Plantago ovata) oder Rhabarberwurzel (Rheum palmatum und Rheum officinale) zu nennen. Meist bevorzugt als pflanzlicher Stoff ist Sennes allein oder in Kombination mit Plantagosamen oder Rhabarberwurzel sowie Sennes Extrakte, reine Sennoside, insbesondere in Form von Sennosid A, Sennosid B, Sennosid C und Sennosid D, und/oder synthetische hergestellte Sennoside, allein oder in Kombination mit Plantagosamen oder Rhabarberwurzel. Die essbare Formulierung enthält den beschichteten partikulären pflanzlichen Stoff regelmäßig in einem Anteil, mit dem der Verbraucher durch Verzehr einer Portion der essbaren Formulierung eine wirksame Menge des partikulären pflanzlichen Stoffs zu sich nehmen kann. Als besonders geeignet kann hier ein Anteil im Bereich von 1 bis 50 Gew.-%, bevorzugt 2 bis 25 Gew.-% und besonders bevorzugt 3 bis 8 Gew.-%, jeweils bezogen auf das Gesamtgewicht der essbaren Formulierung, angegeben werden. Da bei Extrakten, isolierten oder chemisch hergestellten reinen Verbindungen in der Regel eine höhere Konzentration der wirksamen Verbindung vorliegt als bei Pflanzenmaterial, kann die Menge des beschichteten partikulären pflanzlichen Stoffs, der in die essbare Formulierung einbezogen wird, bei Verwendung von Extrakten, isolierten oder chemisch hergestellten reinen Verbindungen als pflanzlicher Stoff auch kleiner sein als 1 Gew.-%, bezogen auf das Gesamtgewicht der essbaren Formulierung.

Der beschichtete partikuläre pflanzliche Stoff ist ein pflanzlicher Stoff, der inklusive der Beschichtung partikulär ist, d.h. die Partikel werden durch den pflanzlichen Stoff und die Beschichtung gebildet. Darüber hinaus ist es im Rahmen der vorliegenden Erfindung bevorzugt, wenn der beschichtete partikuläre pflanzliche Stoff für den Einbezug in die essbare Formulierung nicht in aggregierter oder agglomerierter Form vorliegt, damit sich der Stoff einheitlich in der essbaren Formulierung verteilen lässt. Dadurch wird ein einheitliches Geschmacksempfingen sichergestellt.

Für das Einbeziehen in die erfindungsgemäße essbare Formulierung liegen die pflanzlichen Stoffe in partikulärer Form vor, wobei die Partikel klein genug sein sollten, so dass die Beschichtung während des Verzehrs der Partikel durch Kauen nicht oder nur in vernachlässigbarem Umfang beschädigt werden kann. Entsprechend weist der pflanzliche Stoff eine Partikelgröße von weniger als 2 mm auf, insbesondere weniger als 1 mm und besonders bevorzugt im Bereich 0,05 bis 0,5 mm.

Als im Rahmen der vorliegenden Erfindung besonders geeignete niedermolekulare hydrophobe Substanz sind Glyceryldipalmitostearat, Carnaubawachs und Bienenwachs oder Gemische davon zu nennen, von denen Glyceryldipalmitostearat als bevorzugt angegeben werden kann. Dabei kann Glyceryldipalmitostearat entweder allein oder gegebenenfalls auch im Gemisch mit Bienenwachs und/oder Carnaubawachs eingesetzt werden. Die genannten Substanzen weisen den Vorteil auf, dass sie natürlichen oder naturnahen Ursprungs sind, was vom Verbraucher bei Nahrungsmitteln als vorteilhaft angesehen wird.

Der Anteil der niedermolekularen hydrophoben Substanz, bezogen auf das Gesamtgewicht des beschichteten partikulären pflanzlichen Stoffs liegt im Bereich von 1 bis 20 Gew.-%, bevorzugt im Bereich von 3 bis 15 Gew.-% und weiter bevorzugt im Bereich von 5 bis 12 Gew.-%.

In einer besonders bevorzugten Ausführungsform der vorliegenden Erfindung enthält die essbare Formulierung ein mit Glyceryldipalmitostearat oder einem Gemisch aus Glyceryldipalmitostearat sowie Bienenwachs und/oder Carnaubawachs beschichtetes Sennespulver.

Neben den vorstehend erwähnten essentiellen Bestandteilen kann die erfindungsgemäße essbare Formulierung weitere Bestandteile enthalten, um z.B. die verdauungsfördernde Wirkung weiter zu verbessern.

Ein bevorzugt einzubeziehender weiterer Bestandteil sind z.B. Alditole, die die verdauungsfördernde Wirkung der essbaren Formulierung weiter erhöhen. Alditole, die auch als Polyole oder Zuckeralkohole bezeichnet werden, zeigen eine abführende Wirkung. Alditole kommen auf natürliche Art in Pflanzen vor und können aus diesen extrahiert werden, z.B. Mannitol aus Seegras oder Sorbitol aus Maissirup. Zuckeralkohole wie Mannitol (Mannit) werden durch Reduzierung von Fructose hergestellt. Wenn die erfindungsgemäße essbare Formulierung Alditole enthält, so liegen diese zweckmäßig in einem Anteil im Bereich von 2 bis 25 Gew.-% und insbesondere 5 bis 15 Gew.-% vor.

Ein weiterer bevorzugt in die erfindungsgemäße essbare Formulierung einzubeziehender Bestandteil sind Magnesiumsalze. Magnesium ist ein essentieller Mineralstoff für den Körper, den er nicht selbst herstellen kann und der mit der Nahrung von außen zugeführt werden muss. Magnesium aktiviert über 300 Enzyme. Die in Nahrungsmitteln enthaltenen Magnesiumsalze müssen im Magen-Darm-Trakt erst aufgespalten werden. Durch diese Aufspaltung wird ein freies Magnesium-Ion zur Verwertung freigesetzt und an eine Aminosäure gebunden oder passiv absorbiert.

Ein im Kontext der Erfindung besonders bevorzugtes Magnesiumsalz ist Magnesiumglycinat. Magnesiumglycinat ist besonders gut verträglich und liegt in einer Form vor, in der das Magnesium bereits an die Aminosäure Glycin gebunden ist. Dadurch kann es direkt und schnell aufgenommen und noch schneller verstoffwechselt werden. Falls die erfindungsgemäße essbare Formulierung Magnesiumglycinat enthält, so liegt dieses bevorzugt mit einem Anteil von 1 bis 15 Gew.-% und weiter bevorzugt mit einem Anteil von 3 bis 8 Gew.-% vor. Neben oder alternativ zu Magnesiumglycinat können auch wasserlösliche Magnesiumsalze wie insbesondere Magnesiumcitrat, Magnesiumsulfat und/oder Magnesiumchlorid in die erfindungsgemäße essbare Formulierung einbezogen werden. Für diese kann ein Anteil von 0,5 bis 10 Gew.-% und insbesondere 2 bis 5 Gew.-% als günstig angegeben werden.

Die erfindungsgemäße essbare Formulierung kann auch Bestandteile enthalten, die die Auflösung der niedermolekularen hydrophoben Substanz im Darm beschleunigen. Als besonders geeignet sind hier Calciumcitrat und/oder Kaliumsorbat zu nennen, die in einem Anteil 0,5 bis 10 Gew.-% und insbesondere 2 bis 5 Gew.-% in der essbaren Formulierung vorliegen können.

Ein weiterer verdauungsfördernder Bestandteil, den die erfindungsgemäße essbare Formulierung enthalten kann, ist Inulin. Zugesetztes Inulin wirkt als präbiotischer Nahrungszusatzstoff, da das abbauende Enzym Inulinase im Enddarm durch Bakterien kurzkettige erwünschte Fettsäuren aufbaut. Als günstiger Anteil für Inulin in der erfindungsgemäßen essbaren Formulierung kann ein Anteil von 0,5 bis 10 Gew.-% und insbesondere 2 bis 5 Gew.-% angegeben werden.

Schließlich kann die erfindungsgemäße essbare Formulierung ein oder mehrere Gewürze mit verdauungsfördernder Wirkung erhalten. Solche Gewürze sind z.B. Kümmel, Senf, Anis und Fenchel. Sofern Gewürze mit verdauungsfördernder Wirkung in die erfindungsgemäße Formulierung einbezogen werden, sind diese zweckmäßig in einem Anteil von 0,5 bis 10 Gew.-% und insbesondere 2 bis 5 Gew.-% in diesen enthalten.

Abschließende sei erwähnt, dass die essbare Formulierung zusätzlich zu den vorgenannten Bestandteilen noch weitere übliche Nahrungszusätze, wie Antioxidantien, Säuerungsmittel, Vitamine oder Aromastoffe, enthalten kann. Auch das Einbeziehen von pflanzlichen Stoffen der vorgenannten Art, z.B. Rhabarberwurzeln, die nicht beschichtet sind, ist möglich, für diese Zusätze wäre aber nicht gewährleistet, dass die in diesen pflanzlichen Stoffen enthaltenen verdauungsfördernden Wirkstoffe unzersetzt in den Darm gelangen.

Die hydrophob beschichteten partikulären pflanzlichen Stoffe lassen sich in beliebige essbare Formulierungen integrieren, in denen die Beschichtung nicht durch andere Inhaltsstoffe der Formulierung beschädig oder aufgelöst wird. So ist es z.B. sinnvoll, wenn die essbare Formulierung keine wesentlichen Anteile an flüssigen Fetten enthält, die die hydrophobe Beschichtung auflösen können. Eine besonders geeignete essbare Formulierung im Kontext der vorliegenden Erfindung ist eine Fruchtpastenformulierung, da diese einfach zu portionieren ist und auch als Snack "nach dem Essen" konsumiert werden kann. Demzufolge betrifft ein weiterer Aspekt der vorliegenden Erfindung eine essbare Formulierung, die als Fruchtpastenformulierung vorliegt.

Die "Früchte", auf denen die Fruchtpastenformulierung beruht, unterliegen keinen relevanten Beschränkungen. Als besonders geeignet gelten im Kontext der Erfindung aber Fruchtpastenformulierungen aus Früchten, die ausgewählt sind aus der Gruppe umfassend Feigen, Pflaumen, Aprikosen, Artischocken, Ananas, Papaya und Mischungen davon.

Die Fruchtpastenformulierungen können einerseits medizinisch als Abführmittel verwendet werde. Daher betrifft ein weiterer Aspekt der vorliegenden Erfindung eine entsprechende Fruchtpastenformulierungen zur Verwendung als Abführmittel. Alternativ betrifft die vorliegende Erfindung entsprechende Fruchtpastenformulierungen zur Verwendung bei der Herstellung eines Abführmittels.

Andererseits sind die erfindungsgemäßen Fruchtpastenformulierungen auch zu nicht-medizinischen Zwecken verwendbar, wie z.B. als Brotaufstrich oder als Nahrungsergänzungsmittel.

Offenbart wird weiterhin ein Verfahren zur Herstellung von essbaren Formulierungen, wie vorstehend beschrieben, wobei in einem ersten Schritt ein partikuläres pflanzliches Material mit einer niedermolekularen hydrophoben Substanz beschichtet wird, und in einem zweiten Schritt das beschichtete partikuläre pflanzliche Material in eine essbare Formulierung einbezogen wird.

Das pflanzliche Material kann dazu zweckmäßig aus Planzenteilen wie Blättern oder Früchten durch Vermahlen zu einem Pulver gewonnen werden.

Das Beschichten der partikulären pflanzlichen Stoffe kann mit jedem für eine Beschichtung von Partikeln geeigneten Verfahren durchgeführt werden. So ist es z.B. möglich die Partikel in eine flüssige oder gelöste Form der niedermolekularen hydrophoben Substanz einzutauchen und nach dem herausziehen abzukühlen oder zu trocknen. Da es bei einem solchen Vorgehen aber zu einem unerwünschten Agglomerieren der Partikel kommen kann, wird das Beschichten der pflanzlichen Partikel im Rahmen der vorliegenden Erfindung bevorzugt mit Hilfe eines Sprühtrocknungs- oder Wirbelbettverfahrens durchgeführt. Dabei wird auf ein Wirbelbett der zu beschichtenden Partikel ein Strom aus gelöstem oder verflüssigtem/geschmolzenen hydrophoben Matrixmaterial aufgebracht. Im Rahmen der vorliegenden Erfindung ist die Verwendung eines geschmolzenen hydrophoben Matrixmaterials für das Sprühtrocknungs- oder Wirbelbettverfahren wegen der Vermeidung von Lösungsmittelausdünstungen (wie Ethanol) bevorzugt. Die beschichteten partikulären pflanzlichen Stoffe werden anschließend in eine essbare Formulierung einbezogen, was z.B. durch Einrühren oder Einkneten möglich ist. Die so hergestellten Formulierungen können anschließend portioniert und verpackt oder anderweitig für einen Verkauf konfektioniert werden.

Die erfindungsgemäßen essbaren Formulierungen realisieren vielfältige Vorteile. So sind sie über lange Zeit stabil und weisen gute organoleptische Merkmale, wie einen ansprechenden Geschmack und Geruch, auf. Durch die hydrophobe Beschichtung sind die Wirkstoffe vor dem sauren Milieu des Magens geschützt, so dass sie dort nicht zerstört werden. Somit können die pflanzlichen Stoffe gezielt in den Darm transportiert werden und dort ihre Wirkung entfalten.

Die beschichteten partikulären pflanzlichen Stoffe sind auf Grund der einstellbaren Konsistenz für die essbaren Formulierungen für den Einsatz in verdauungsfördernden Fruchtpasten geeignet, die als Abführmittel, Nahrungsergänzungsmittel oder Brotaufstrich verwendet werden können.

Im Folgenden wir die vorliegende Erfindung anhand eines Beispiels noch näher erläutert:
Beispiel:
Gemahlene Sennesblätter (100 - 200 µm) wurden in einem Wirbelbettverfahren mit Glyceryldipalmitostearat beschichtet. Dazu wurde das Glyceryldipalmitostearat auf 120°C erwärmt und bei einem Zerstäuberdruck von 2 bar in einen Sprühraum eingedüst, in dem sich die auf 45°C erwärmten gemahlenen Sennesblätter befanden. Die Beschichtungsmenge an Glyceryldipalmitostearat wurde auf einen Anteil von 7 - 12 Gew.-% eingestellt. Für die Beschichtung wurde die Flughöhe der Partikeln im Wirbelbettcoater so eingestellt, dass ein Agglomerieren der Partikel weitestgehend unterdrückt wird.

Das so erhaltene mit Glyceryldipalmitostearat beschichtete Sennespulver wurde mit einem Anteil von 7 Gew.-% in eine vorgefertigte Feigenpaste eingerührt, portioniert und abgepackt.

## Patentansprüche

1. Essbare Formulierung, umfassend einen mit einer niedermolekularen hydrophoben Substanz, die ein Molekulargewicht von < 1000 g/mol aufweist, beschichteten partikulären pflanzlichen Stoff, der Wirkstoffe zur Verdauungsförderung enthält, wobei der partikuläre pflanzliche Stoff eine Partikelgröße von weniger als 2 mm aufweist und inklusive der Beschichtung partikulär ist, der partikuläre pflanzliche Stoff in Form von Sennes, insbesondere in Form von Sennesblättern, Sennesextrakt, reinen Sennosiden, Rhabarberwurzeln oder Rhabarberwurzelextrakt vorliegt und der beschichtete partikuläre pflanzliche Stoff einen Anteil an der niedermolekularen hydrophoben Substanz von 1 bis 20 Gew.-% enthält, und wobei die essbare Formulierung so formuliert ist, dass die Beschichtung des partikulären pflanzlichen Stoffs nicht durch andere Inhaltsstoffe der Formulierung beschädigt oder aufgelöst wird.

2. Essbare Formulierung nach Anspruch 1, **dadurch gekennzeichnet, dass** der partikuläre pflanzliche Stoff in Form von Sennes in Kombination mit Plantagosamen vorliegt.

3. Essbare Formulierung nach Anspruch 1, **dadurch gekennzeichnet, dass** die essbare Formulierung einen Anteil des mit einer niedermolekularen hydrophoben Substanz beschichteten partikulären pflanzlichen Stoffs von 1 bis 50 Gew.-%, bevorzugt von 2 bis 25 Gew.-% und besonders bevorzugt von 3 bis 8 Gew.-% enthält.

4. Essbare Formulierung nach einem der Ansprüche 1 oder 2, **dadurch gekennzeichnet, dass** die niedermolekulare hydrophobe Substanz in Form von Glyceryldipalmitostearat, Carnaubawachs, Bienenwachs oder einem Gemisch davon, bevorzugt in Form von Glyceryldipalmitostearat vorliegt.

5. Essbare Formulierung nach einem der vorstehenden Ansprüche, **dadurch gekennzeichnet, dass** der beschichtete partikuläre pflanzliche Stoff einen Anteil an der niedermolekularen hydrophoben Substanz von 3 bis 15 Gew.-% und bevorzugt 5 bis 12 Gew.-% enthält.

6. Essbare Formulierung nach einem der vorstehenden Ansprüche, **dadurch gekennzeichnet, dass** sie weiterhin Alditole, bevorzugt mit einem Anteil von 2 bis 25 Gew.-% und besonders bevorzugt mit einem Anteil von 5 bis 15 Gew.-% enthält.

7. Essbare Formulierung nach einem der vorstehenden Ansprüche, **dadurch gekennzeichnet, dass** sie weiterhin Magnesiumglycinat, bevorzugt mit einem Anteil von 1 bis 15 Gew.-% und besonders bevorzugt mit einem Anteil von 3 bis 8 Gew.-% enthält.

8. Essbare Formulierung nach einem der vorstehenden Ansprüche, **dadurch gekennzeichnet, dass** sie weiterhin ein wasserlösliches Magnesiumsalz, ausgewählt aus der Gruppe umfassend Magnesiumcitrat, Magnesiumsulfat und Magnesiumchlorid, enthält.

9. Essbare Formulierung nach Anspruch 7, **dadurch gekennzeichnet, dass** der Anteil an wasserlöslichem Magnesiumsalz 0,5 bis 10 Gew.-% und bevorzugt 2 bis 5 Gew.-% beträgt.

10. Essbare Formulierung nach einem der vorstehenden Ansprüche, **dadurch gekennzeichnet, dass** sie weiterhin Calciumcitrat und/oder Kaliumsorbat umfasst, bevorzugt mit einem Anteil von 0,5 bis 10 Gew.-% und weiter bevorzugt von 2 bis 5 Gew.-%.

11. Essbare Formulierung nach einem der vorstehenden Ansprüche, **dadurch gekennzeichnet, dass** sie weiterhin Inulin enthält, bevorzugt mit einem Anteil von 0,5 bis 10 Gew.-% und weiter bevorzugt von 2 bis 5 Gew.-%.

12. Essbare Formulierung nach einem der vorstehenden Ansprüche, **dadurch gekennzeichnet, dass** sie weiterhin mindestens ein Gewürz ausgewählt aus der Gruppe umfassend Kümmel, Senf, Anis und Fenchel enthält, bevorzugt mit einem Anteil von 0,5 bis 10 Gew.-% und weiter bevorzugt 2 bis 5 Gew.-%.

13. Essbare Formulierung nach einem der vorstehenden Ansprüche, **dadurch gekennzeichnet, dass** sie als Fruchtpastenformulierung vorliegt.

14. Essbare Formulierung nach Anspruch 13, **dadurch gekennzeichnet, dass** die Fruchtpastenformulierung auf Früchten, ausgewählt aus der Gruppe umfassend Feigen, Pflaumen, Aprikosen, Artischocken, Ananas, Papaya und Mischungen davon, basiert.

15. Essbare Formulierung nach Anspruch 13 oder 14 zur Verwendung als Abführmittel.

16. Verwendung einer essbaren Formulierung nach Anspruch 13 oder 14 als Brotaufstrich oder Nahrungsergänzungsmittel.

## Claims

1. An edible formulation, comprising a particulate plant substance coated with a low-molecular hydrophobic substance which has a molecular weight of < 1000 g/mol, which substance contains active ingredients for aiding digestion, wherein the particulate plant substance has a particle size of less than 2 mm and including the coating is particulate, the particulate plant substance is present in the form of senna, espcially in the form of senna leaves, senna extract, pure sennosides, rhubarb roots or rhubarb root extract, and the coated particulate plant substance has a proportion of the low-molecular hydrophobic substance of 1 to 20 wt.%, and wherein the edible formulation is formulated such that the coating of the particulate plant substance is not damaged or dissolved by other ingredients of the formulation.

2. An edible formulation according to Claim 1, **characterised in that** the particulate plant substance is present in the form of senna in combination with plantago seeds.

3. An edible formulation according to Claim 1, **characterised in that** the edible formulation contains a proportion of the particulate plant substance coated with a low-molecular hydrophobic substance of 1 to 50 wt.%, preferably of 2 to 25 wt.%, and espcially preferably of 3 to 8 wt.%.

4. An edible formulation according to one of Claims 1 or 2, **characterised in that** the low-molecular hydrophobic substance is present in the form of glyceryl dipalmitostearate, carnauba wax, beeswax or a mixture thereof, preferably in the form of glyceryl dipalmitostearate.

5. An edible formulation according to one of the preceding claims, **characterised in that** the coated particulate plant substance contains a proportion of the low-molecular hydrophobic substance of 3 to 15 wt.% and preferably 5 to 12 wt.%.

6. An edible formulation according to one of the preceding claims, **characterised in that** it furthermore contains alditols, preferably in a proportion of 2 to 25 wt.% and espcially preferably in a proportion of 5 to 15 wt.%.

7. An edible formulation according to one of the preceding claims, **characterised in that** it furthermore contains magnesium glycinate, preferably in a proportion of 1 to 15 wt.% and espcially preferably in a proportion of 3 to 8 wt.%.

8. An edible formulation according to one of the preceding claims, **characterised in that** it furthermore contains a water-soluble magnesium salt, selected from the group comprising magnesium citrate, magnesium sulphate and magnesium chloride.

9. An edible formulation according to Claim 7, **characterised in that** the proportion of water-soluble magnesium salt is 0.5 to 10 wt.% and preferably 2 to 5 wt.%.

10. An edible formulation according to one of the preceding claims, **characterised in that** it furthermore comprises calcium citrate and/or potassium sorbate, preferably in a proportion of 0.5 to 10 wt.% and more preferably of 2 to 5 wt.%.

11. An edible formulation according to one of the preceding claims, **characterised in that** it furthermore contains inulin, preferably in a proportion of 0.5 to 10 wt.% and more preferably of 2 to 5 wt.%.

12. An edible formulation according to one of the preceding claims, **characterised in that** it furthermore contains at least one spice selected from the group comprising caraway, mustard, aniseed and fennel, preferably in a proportion of 0.5 to 10 wt.% and more preferably 2 to 5 wt.%.

13. An edible formulation according to one of the preceding claims, **characterised in that** it is present as a fruit paste formulation.

14. An edible formulation according to Claim 13, **characterised in that** the fruit paste formulation is based on fruits, selected from the group comprising figs, plums, apricots, artichokes, pineapple, papaya and mixtures thereof.

15. An edible formulation according to Claim 13 or Claim 14 for use as a laxative.

16. Use of an edible formulation according to Claim 13 or Claim 14 as a spread or dietary supplement.

## Revendications

1. Formulation comestible, comprenant une substance végétale particulaire enrobée d'une substance hydrophobe de faible poids moléculaire, qui présente un poids moléculaire de < 1000 g/mol, et contenant des principes actifs pour favoriser la digestion, la substance végétale particulaire présentant une taille de particule inférieure à 2 mm et étant particulaire, y compris le revêtement, la substance végétale particulaire se présentant sous la forme de séné, en particulier de feuilles de séné, d'extrait de séné, de sennosides purs, de racines de rhubarbe ou d'extrait de racine de rhubarbe et la substance végétale particulaire enrobée comprenant une proportion de la substance hydrophobe de faible poids moléculaire de 1 à 20 % en poids, et la formulation comestible étant formulée de telle sorte que l'enrobage de la substance végétale particulaire n'est pas détérioré ou dissous par d'autres ingrédients de la formulation.

2. Formulation comestible selon la revendication 1, **caractérisée en ce que** la substance végétale particulaire se présente sous la forme de séné en combinaison avec des graines de plantain.

3. Formulation comestible selon la revendication 1, la formulation comestible étant **caractérisée en ce qu'**elle comprend une proportion de la substance végétale particulaire enrobée par la substance hydrophobe de faible poids moléculaire de 1 à 50 % en poids, de préférence 2 à 25 % en poids, et de manière particulièrement préférée de 3 à 8 % en poids.

4. Formulation comestible selon l'une des revendications 1 ou 2, **caractérisée en ce que** la substance hydrophobe de faible poids moléculaire se présente sous la forme de dipalmitostéarate de glycéryle, de cire de carnauba, de cire d'abeilles, ou d'un mélange de ceux-ci, de préférence sous la forme de dipalmitostéarate de glycéryle.

5. Formulation comestible selon l'une des revendications précédentes, **caractérisée en ce que** la substance végétale particulaire enrobée comprend une proportion de la substance hydrophobe de faible poids moléculaire de 3 à 15 % en poids et de préférence de 5 à 12 % en poids.

6. Formulation comestible selon l'une des revendications précédentes, **caractérisée en ce qu'**elle contient en outre des alditols, de préférence en une proportion de 2 à 25 % en poids et de manière particulièrement préférée en une proportion de 5 à 15 % en poids.

7. Formulation comestible selon l'une des revendications précédentes, **caractérisée en ce qu'**elle contient en outre du glycinate de magnésium, de préférence en une proportion de 1 à 15 % en poids et de manière particulièrement préférée en une proportion de 3 à 8 % en poids.

8. Formulation comestible selon l'une des revendications précédentes, **caractérisée en ce qu'**elle contient en outre un sel de magnésium hydrosoluble, choisi dans le groupe comprenant le citrate de magnésium, le sulfate de magnésium et le chlorure de magnésium.

9. Formulation comestible selon la revendication 7, **caractérisée en ce que** la proportion de sel de magnésium hydrosoluble est de 0,5 à 10 % en poids et de préférence de 2 à 5 % en poids.

10. Formulation comestible selon l'une des revendications précédentes, **caractérisée en ce qu'**elle contient en outre du citrate de calcium et/ou du sorbate de potassium, de préférence en une proportion de 0,5 à 10 % en poids et en outre de manière encore mieux préférée de 2 à 5 % en poids.

11. Formulation comestible selon l'une des revendications précédentes, **caractérisée en ce qu'**elle contient en outre de l'inuline, de préférence en une proportion de 0,5 à 10 % en poids, et de manière encore mieux préférée de 2 à 5 % en poids.

12. Formulation comestible selon l'une des revendications précédentes, **caractérisée en ce qu'**elle contient en outre au moins une épice choisie dans le groupe constitué du cumin, de la moutarde, de l'anis et du fenouil, de préférence en une proportion de 0,5 à 10 % en poids, et de manière encore mieux préférée de 2 à 5 % en poids.

13. Formulation comestible selon l'une des revendications précédentes, **caractérisée en ce qu'**elle se présente sous forme d'une formulation de pâtes de fruits.

14. Formulation comestible selon la revendication 13, **caractérisée en ce que** la formulation de pâtes de fruits est basée sur des fruits, choisis dans le groupe comprenant les figues, les prunes, les abricots, les artichauts, les ananas, la papaye et des mélanges de ceux-ci.

15. Formulation comestible selon la revendication 13 ou 14 pour utilisation en tant que laxatif.

16. Utilisation d'une formulation comestible selon la revendication 13 ou 14 comme pâte à tartiner ou complément alimentaire.
